# EUROPEAN PATENT SPECIFICATION

(11) **EP 0 532 896 B1**
(45) Date of publication and mention of the grant of the patent: **19.03.1997**
(21) Application number: 92113569.5
(22) Date of filing: 10.08.1992
(51) Int. Cl.: C08G 59/22, C08G 59/00, C07D 303/00, H01L 23/16

(54) **Cleavable diepoxide for removable device protection in electronic packaging**
Spaltbare Diepoxyde als entfernbarer Vorrichtungsschutz für elektronisches Gehäuse
Diépoxyde clivable comme protection d'appareillage amovible pour des composants électroniques

(30) Priority: 05.09.1991 US 755253
(43) Date of publication of application: 24.03.1993
(73) Proprietor: International Business Machines Corporation, Armonk, N.Y. 10504 (US)
(72) Inventor: Buchwalter, Stephen L., Wappingers Falls, NY 12590 (US); Kosbar, Laura L., Mohegan Lake, NY 10547 (US); Newman, Bert H., Carmel, NY 10512 (US); Pompeo, Frank L., Walden NY 12586 (US)
(74) Representative: Schäfer, Wolfgang, Dipl.-Ing.

(56) References cited:
- WO-A-86/03145
- GB-A- 0 865 340
- US-A- 3 255 215
- DATABASE WPIL Derwent Publications Ltd., London, GB; AN 88-300282

## Description

The present invention relates to a cleavable epoxy resin composition, its application in a structure with electronic devices and a method for its removal.

In the field of electronics, chips mounted onto substrates are often encapsulated with a cured epoxy which is insoluble.

Epoxy resins comprise a family of thermoset plastics whose properties have been found to be particularly well suited to device protection applications in electronic packaging. The positive attributes of epoxy resins as materials for encapsulation and other protection applications of the aforementioned devices are well known. For many packaging applications, however, replacement of one or more chips, known as rework, is often necessary after package assembly is complete.

Epoxies are applied in unreacted or partly reacted form, which means that the viscosity of the material can be quite low, providing ease of processing and good wetting of device surfaces. Curing of the material at some moderate temperature then generates the rigid epoxy matrix desired for device protection. Filler additives are generally included in the matrix to reduce the coefficient of thermal expansion (CTE) of the cured material to minimize stresses induced by the difference in expansion of the plastic and the device during thermal cycling. As described in Handbook of Epoxy Resins, H. Lee and K. Neville, McGraw Hill, (1967), fully cured epoxy resins, with or without filler, are heavily crosslinked insoluble network polymers. As a result, removal of such epoxy materials from electronic packages without damage to the circuitry or devices has been and is virtually impossible.

U.S. Patent 4,159,221 discloses a method for packaging electronic circuits and a method for providing a hermetically sealed electronic circuit package. The reference discloses that the sealant used is an epoxy which is asserted to be readily soluble after being cured. The epoxy described in the reference is a conventional commercial bis-phenol A diglycidyl ether crosslinked with diaminodiphenylsulfone. The amount of the crosslinking agent is quite low since according to Lee and Neville (H. Lee and K. Neville, Handbook of Epoxy Resins, McGraw-Hill, 1967, pp. 8-8, 8-9) stoichiometric amounts of diaminodiphenylsulfone with bis-phenol A diglycidyl ether (DGEBA) would be 33 parts/100 parts DGEBA. The amount used in the reference is only 10 parts/100. It has been determined that curing with aromatic diamines such as diaminodiphenylsulfone is very sluggish so without any stipulation as to cure temperature given in the reference with the small amount of cross-linking agent being used one has to proceed from the assumption that the curing reaction is not complete. In accordance with the present invention, a properly cured, conventional epoxy will not be soluble. A partially cured epoxy, such as is disclosed in the reference may be soluble and may serve some function in their application but will not have the physical properties needed for the present invention, i.e. reinforcement of solder interconnections and other encapsulation applications.

British Patent 865,340 discloses diepoxides having linear ketal or acetal linkages, a cyclic anhydride curing agent and optionally an accelerator, such as a tertiary amine. There is no mention of the possible utility of such diepoxides that are cleavable in acid. In fact, stability of the cured ketal diepoxide in strong acid is claimed in Example 16 of this patent. Accordingly, this patent does not render the present invention obvious.

Sastri and Tesoro (J. Appl. Polym. Sci., 1990, 39 1425-1457) have published experiments on reversible crosslinking of epoxy resins having a disulfide link. Dissolution of their epoxies require pulverization of the epoxy to a powder and many hours exposure to oxidizing agents. For these reasons, these epoxies are not suitable for the purposes of the present invention nor do the structures disclosed render the present invention obvious.

U.S. Patent 2,895,962 discloses diepoxides containing cyclic acetal groups. The introduction in this reference discusses the expectation that acetals will be sensitive to degradation by acids and claims that the cyclic acetals in fact are surprisingly resistant to acids. There is no mention of the possible utility of an epoxide that is cleavable in acid nor is such an acid disclosed. Experimental tests have confirmed that cyclic acetals are not cleavable. For example, under conditions similar to that used to cleave the preferred compositions of the present invention after curing, a commercial epoxide cured the same way and containing a cyclic acetal group is unaffected. Even hydrochloric acid does not degrade the epoxides containing the cyclic acetal group.

Accordingly, the cyclic acetals disclosed in this and the other patents noted below are not suitable for the purpose set forth herein and do not render the invention obvious.

U.S. Patent 3,264,235 discloses soluble reaction products of aromatic epoxides and silicates. These compounds are not pertinent relative to the present invention.

U.S. Patents 3,759,954; 3,879,422 and 3,956,317 all disclose compositions of matter covering diepoxides of diketals and diacetals which are cyclic in structure. The compounds are cured to epoxy matrices which are not cleavable.

U.S. Patent 4,153,586, is not pertinent as it refers to reaction products of epoxides which are not suitable for curing to rigid matrices by reaction with crosslinking agents such as cyclic anhydrides. Although this patent discloses ketal and acetal diepoxides there is no mention of cleavability of the epoxides or their utility as a removable cured epoxy material.

In US Patent 3,255,215 epoxy acetals are disclosed. They are useful in the preparation of expoxy resins. They can form useful compositions when hardened with polyamides, polyacids, anhydrides and the like. The acetal starting materials and products for forming the epoxy acetals are hydrolyzable in the presence of strong acids and water.

In DD Patent 257 435 A1 a method for producing acid cleavable epoxy addition polymers is disclosed wherein diepoxides are reacted with acetal compounds. The resulting polymer being useful in electronics is cleavable in an acidic solvent system containing water and also soluble in an organic solvent or solvent mixture.

A cleavable epoxy resin composition, its application and the method for its removal which form the invention are disclosed in claims 1, 12 and 11.

The present invention discloses a means of achieving a crosslinked epoxy matrix maintaining all the advantages of previously known epoxy materials that is also easily removable if the need arises.

In the present invention use of soluble diepoxides with acetal or ketal linkages permits encapsulating electronic chips, testing them to ascertain that the product satisfies the manufacturing specification and if needed, removing the chips for rework by dissolving the epoxy without destroying the chip or substrate.

The solvent system used to remove the cured diepoxide is another aspect of the present invention. Certain solvent systems have been found to attack the metallization or the circuit card material. The solvent systems used in accordance with the present invention provide a reworkable encapsulant process compatible with manufacturing and environmental concerns.

Pursuant to the present invention, a straightforward two-step synthesis is carried out to prepare a diepoxide having linear ketal or acetal linkages.

Pursuant to the present invention, the combination of the low viscosity of the epoxy precursor mixture and the high modulus and relatively low CTE of the cured matrix has been found to be advantageous for a particularly important device protection application. A particularly useful embodiment is the protection of solder ball interconnections of chips to ceramic or other substrates. A low viscosity precursor mixture can efficiently and completely fill the spaces between chip and substrate and envelop solder ball interconnections with epoxy. Cured epoxy generally provides remarkable enhancement of the solder ball interconnections life with respect to times to failure in thermal cycling. Because of the ability, in view of this reinforcement, to mount large chips that would otherwise show unacceptable failure rates, this application for epoxy materials generates substantial interest. Wide application of this reinforcement, however, would require that the epoxy be removable in order to allow rework of a single chip in a multi-chip module. As stated above, cured epoxy resins are known for their intractability.

The present invention is based on the recognition that the intractability of the cured epoxy matrix may result from the tetrafunctional nature of the diepoxide starting material. The diepoxide forms a network of macro-scale dimensions with a difunctional hardener such as a cyclic anhydride. Without degradation, such a network cannot dissolve in any solvent. If the two epoxy groups of the diepoxide can be connected by a cleavable bond or link, then cleavage of such links would convert the network immediately to a collection of small molecules, which will be soluble.

In searching for appropriate structures that can serve as links for the diepoxide, the following criteria are important.
1. The link should be stable under conditions to which the cured matrix would normally be exposed;
2. The link should be readily cleaved under specific conditions;
3. The link should be unreactive in the curing reaction of the epoxy matrix;
4. A practical synthesis of the diepoxide containing the link should be available.

The ketal and acetal groups have been identified as candidates meeting the above criteria.

As disclosed in Advanced Organic Chemistry, J. March, 3rd Edition, Wiley Interscience (1985), pp. 329-331, the known organic chemistry of ketals and acetals indicates that they are exceedingly stable to hydrolysis in the absence of acids, but break down readily in acid, even weak acids. Ketals and acetals are not subject to reactions similar to those of epoxy groups, and thus a ketal link should not be affected by the curing reaction of the epoxy matrix.

Acetals can be hydrolyzed in acidic aqueous solutions, but they are also susceptible to trans-etherification under acidic conditions. It then becomes possible to use an alcohol as both the solvent and the reactant, removing the necessity of adding water to the system.

Other advantageous embodiments of the inventive composition and the inventive structure are disclosed in the subclaims.

The invention will become more apparent from the following detailed description taken in conjunction with the accompanying drawings.
- Fig. 1: is is the preferred structure of diepoxide containing linear ketal or acetal links.
- Fig. 2: is is the generalized structure of diepoxides containing linear acetal and ketal links.
- Fig. 3: depicts various diepoxides that can be used in accordance with the present invention.

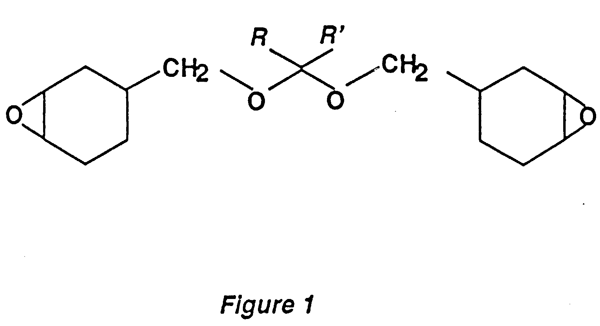

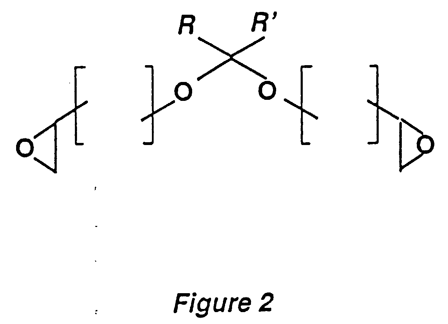

The ketal and acetal diepoxides of the present invention are synthesized and then mixed with a cyclic anhydride, an amine promoter, and, optionally, a flexibilizer and an inorganic filler such as silica. The preferred ketal/acetal diepoxide structure is shown in Figure 1, in which R and R' can be any combination of hydrogen, methyl, ethyl, propyl, iso-propyl, butyl, iso-butyl, other alkyl, phenyl, benzyl, substituted phenyl or substituted benzyl. Substituents on the phenyl or benzyl can be at one or more of the available aromatic positions and can be a halogen such as chlorine, bromine, or fluorine, a nitro group, any alkyl group such as methyl, ethyl, or isopropyl, an alkoxy group such as methoxy, ethoxy, or isopropoxy, an acyl such as acetyl or benzoyl, or any of the family of aromatic substituent groups well known in organic chemistry. In addition to the structure of Figure 1, the diepoxide can be any diepoxide in which the two epoxide groups are connected by an acetal or ketal as in Figure 2, in which R and R' are as in Figure 1.

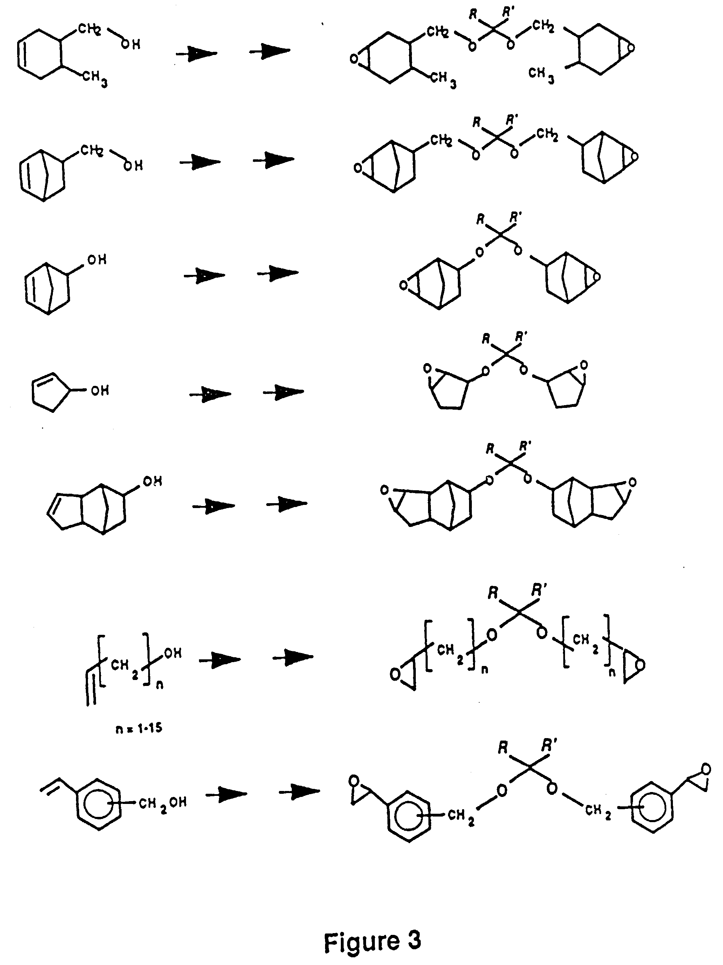

The epoxy structures suitable for use in this invention are those derived from olefinic alcohols. The alcohol functionality preferably is an aliphatic primary or secondary alcohol group, most preferably a primary alcohol group, and must be suitable for reaction with an aldehyde or ketone to form an acetal or ketal. The olefinic funtionality is preferably an aliphatic double bond, most preferably a mono-substituted or a 1,2-disubstituted double bond, and must be suitable for epoxidation with an epoxidation reagent known in the art, such as peracetic acid, perbenzoic acid, metachloroperbenzoic acid, potassium peroxymonosulfate, and the like. Alternative olefinic alcohols and the acetal or ketal diepoxides derived from them are shown in Figure 3, in which R and R' are as in Figure 1.

The cyclic anhydride can be any of the well known anhydride curing agents (see for example Lee and Neville, Chapter 12) including hexahydrophthalic anhydride, methyl-hexahydrophthalic anhydride, nadic methyl anhydride, and maleic anhydride.

The amine promoter is a tertiary amine or aromatic amine selected from the group benzyldimethylamine, triethylamine, pyridine, imidazole, propoxylated imidazole, 1,4-diazabicyclo[2.2.2]octane.

The optional flexibilizer can be any of the well known materials used for this purpose such as the Union Carbide ERL-4350, LHT-34 or LHT-240 or the B.F. Goodrich butadiene-acrylonitrile copolymers sold under the tradename HYCAR®.In addition, any polyetherdiol or polyesterdiol can be used as a flexibilizer including polyethylene glycol, polypropylene glycol, poly(caprolactone)diol, or poly(oxybutylene)diol.

Encapsulants are commonly filled with an inorganic powder to reduce the coefficient of thermal expansion. Suitable filler materials include alumina, silica, and the like. The preferred filler is a highly purified form of silica with particle size of 25 micrometers or less. The amount of filler may vary but is preferred in the range 50-70% on a weight basis.

The epoxy formulations are cured by heating at 90-200°C for 1-6 hours, preferably 100-150°C for about 2 hours to form a hard tack-free solid. It is important that the cured resin resulting from the cleavable diepoxide is not adversely affected by the environment to which it is likely to be exposed. As an aggressive test for moisture resistance, the ketal diepoxide (Figure 1, R=R'=methyl) formulation was exposed to boiling water along with a commercial non-cleavable formulation based on Union Carbide ERL-4221. The ketal diepoxide formulation showed a slight surface blush and no loss of adhesion to glass; the commercial formulation showed no blush but lost adhesion to the glass side. Under the long-term exposure to elevated temperature and humidity (85°C/81%R.H.), the ketal diepoxide formulation showed significant softening. The corresponding acetal diepoxide (Figure 1, R=methyl, R'=hydrogen) formulation was unchanged after 4 weeks in elevated temperature and humidity testing.

Finally, it is obvious that for the new resin to be useful as a reworkable material for device protection, conditions must be found for its removal. Acetals and ketals are generally easily cleaved in aqueous acid, but in order to dissolve the matrix an organic solvent is also needed. Many mixtures of organic solvents, acid or acids, and water can be used. For the purposes of this invention, suitable acids include organic acids such as acetic acid, propionic acid, chloroacetic acid, benzoic acid and the like; sulfonic acids such as benzenesulfonic acid, p-toluenesulfonic acid, methanesulfonic acid and the like; inorganic acids such as sulfuric acid, phosphoric acid, hydrochloric acid, and the like; and Lewis acids such as boron trifluoride etherate, aluminum chloride, stannic chloride and the like. These structures are exemplary only and are disclosed to illustrate the type of solvents and acids to be used. The following mixtures are given only as examples. The temperature of the solvent mixture can be 25°C or above, but for most rapid dissolution the solvents should be heated to boiling or near boiling. One useful mixture is that of ethanol, acetic acid, and water which is particularly effective in dissolving the cured formulations based on the ketal diepoxide. Other suitable solvent mixtures include a combination of gamma-butyrolactone, water, and phosphoric acid and a combination of butanol, acetic acid and water. Acetals and ketals are also susceptible to trans-etherification under acidic conditions. It then becomes possible to use an alcohol as both the solvent and the reactant, removing the necessity of adding water to the system thereby reducing the likelihood of corrosion of metallic components of the device. Even at refluxing temperatures, the alcohols are also very benign with respect to attack of the dielectric matrix used in circuitry such as FR4 epoxy (FR4 is the trade name of 2,2',6,6' tetrabromobisphenol A diglycidylether homopolymer). For example, a mixture of ethanol and an organic acid such as benzenesulfonic acid, para-toluenesulfonic acid, or methanesulfonic acid can be used to dissolve the cured epoxy based on the acetal diepoxide.

### EXAMPLE 1

This example discloses the preparation of acetone bis-(cyclohex-3-enylmethyl)ketal: 3-cyclohexene-1-methanol (224.3 g, 2.00 moles) was dissolved in a mixture of 2,2-dimethoxypropane (104.2 g, 1.00 moles), cylohexane (330 ml), and p-toluenesulfonic acid (0.1 g). The mixture was heated to reflux through a distillation column, and the cylohexane/methanol azeotrope (bp 54.2°C) was removed over 5 hours. The head temperature gradually increased to 81°C. The yellow pot residue was cooled, and the acid was neutralized with sodium methoxide. The remaining solvent was removed on the rotary evaporator, the residue was distilled under vacuum (bp 99-100°/0.8 mm Hg) to give a 75% yield of the pure diolefinic ketal identified by IR and NMR.

### EXAMPLE 2

This example discloses the preparation of acetone bis-(3,4-epoxycyclohexylmethyl)ketal (ketal diepoxide): Successful epoxidation of the diolefinic ketal required a procedure which did not include exposure of the ketal to acidic conditions. Such a procedure was found in the literature (Curci, R.; Fiorentino, M.; Troisi, L. J. Org. Chem., 1980, 45, 4758-4760) and adapted as follows. The diolefinic ketal (13.48 g, 0.051 moles) was dissolved in dichloromethane (150 ml) and acetone (150 ml) and mixed with a pH 7.5/0.1M phosphate buffer (100 ml and 18-crown-6 (1.5 g) as a phase transfer agent. The mixture was stirred in an ice bath to reduce its temperature to 6°C before the start of the simultaneous addition of a solution of potassium peroxymonosulfate (OXONE, DuPont, 73.8 g in 400 ml deionized water containing 0.09 g of ethylenediaminetetraacetic acid) and a 0.5M KOH solution. Rapid stirring and chilling with ice were continued throughout the addition, and the relative rate of the addition of the two solutions was adjusted to keep the pH of the reaction mixture in the range 7.3-7.9. Addition of the OXONE solution was complete in 2 hours. During an additional 3.5 hours of stirring, the temperature was kept at 6-8°C, and the pH was maintained in the above range by continued slow addition of 1.0M KOH. At the end of this time, additional dichloromethane (100 ml) was stirred in for 5 minutes. The dichloromethane phase was isolated, and the aqueous phase was shaken with additional dichloromethane (100 ml). The combined dichloromethane solutions were then dried over magnesium sulfate, filtered and evaporated to give a 95% yield of liquid diepoxide identified by IR and NMR. Epoxide equivalent weight (weight per epoxide) was 161 (theory 148).

### EXAMPLE 3

This example discloses the preparation of acetaldehyde bis-(cyclohex-3-enylmethyl) acetal: The Ciba procedure (British Patent 865,340, 1959) was followed to prepare the aforementioned composition. A mixture of 3-cyclohexene-1-methanol (75 g, 0.67 moles), mercuric oxide (0.5 g) and boron trifluoride diethyl etherate (0.5 g) was warmed to 55°C before vinyl acetate (30 g, 0.35 moles) was added drop wise with stirring. After the addition (about 2 hours, the solution was held at 60°C for 16 hours then left at ambient temperature for 2 days. After quenching with 28 g sodium carbonate and 125 g ice-cold water, the organic phase was combined with a 50 ml toluene extract of the aqueous phase, dried over sodium sulfate, and filtered. The solvent was removed by rotary evaporation, and the residue was distilled under vacuum at 0.4-0.5 mm. The forerun boiling at < 100°C was discarded. The resultant material (36 g or 43%) was collected at 110-125°C and identified by IR and NMR.

### EXAMPLE 4

This example discloses the preparation of acetaldehyde bis-(3, 4-epoxyclohexylmethyl) acetal (acetal diepoxide): Following the same procedure as for the ketal diepoxide, a quantitive yield of acetal diepoxide was obtained, which was free of olefin by IR and NMR and had an epoxy equivalent weight of 146 (theory 141).

### EXAMPLE 5

This example discloses the preparation of formaldehyde bis-(cyclohex-3-enylmethyl) acetal: The Ciba procedure was followed except that water was removed in a Dean-Stark trap to drive the reaction to completion. Spectra (IR and NMR) confirmed the structure.

### EXAMPLE 6

This example discloses the preparation of formaldehyde bis-(3,4-epoxycyclohexylmethyl) acetal (formal diepoxide): Using the above described epoxidation procedure, a material was obtained which had appreciable unreacted olefin by IR. This was subjected to a repeat epoxidation, resulting in a 61% yield of olefin-free product (IR and NMR). Epoxy equivalent weight was 137 (theory 134).

In example 7 the curing of ketal and acetal diepoxides being defined in claim 1 and of the Union Carbide cyloaliphatic diepoxide ERL-4221 was studied and in examples 8 and 9 the behaviour of the thus obtained cured compositions when treated with water or with a number of acid containing solutions was evaluated where the experiments with the Union Carbide diepoxide provide comparison data.

### EXAMPLE 7

This example provides curing studies: The Union Carbide cycloaliphatic diepoxide ERL-4221 (2.00 g, measured epoxy equivalent weight 141) was mixed with melted hexahydrophthalic anhydride (2.00 g), ethylene glycol (0.02 g) and propoxylated imidazole (Archem AP-5, 0.04 g). The first three components were thoroughly blended before mixing in the imidazole. The mixture was then spotted from a pipet onto microscope slides and cured at 150°C for 2 hours. The spots were hard tack-free solids, as expected for a fully cured epoxy resin. Similar results were obtained with various levels of benzyldimethylamine (BDMA) instead of propoxylated imidazole. A DSC run using BDMA showed a single exotherm with maximum at 146°C.

Similarly, ketal diepoxide (2.16 g) was mixed with hexahydrophthalic anhydride (2.00 g), ethylene glycol (0.02. g) and propoxylated imidazole or benzyldimethlamine (0.04 g). After curing under the same conditions, the result was hard and tack-free as above. A DSC run had a similar exotherm to that of the commercial diepoxide (maximum 141°C).

The acetal diepoxide (1.05 g) was also mixed with the anhydride (1.05 g), ethylene glycol (0.01 g), and BDMA (0.13 g). Its curing behavior was quite similar, hard and tack-free with an exotherm in DSC peaking at 148°C.

The formal diepoxide (0.52 g) was also mixed with the anhydride (0.507 g), ethylene glycol (0.01 g), and BDMA (0.015 g) and cured as above. No significant differences were seen in hardness or DSC (exotherm maximum 148°C).

### EXAMPLE 8

The effect of water on the cured epoxies of Example 7 were compared. Both the sample derived from the ketal diepoxide and that derived from Union Carbide ERL-4221 (Control) were soaked in water for 4 days with no visual change in the spots. Both were also heated in water at 60°C for 1 hour, again with no visual change. The samples were boiled in water for 1 hour. The ketal diepoxide sample showed a slight surface blush but not loss of adhesion to the glass slide; the Control was not blushed but no longer adhered to the glass. Finally, the samples soaked in water at room temperature for 4 days were immersed in boiling water for an hour. The new resin was heavily blushed but suffered no apparent loss of adhesion; the Control was not blushed but again no longer was adhering to the glass.

Cured samples of the ketal and acetal diepoxide formulations were also exposed to elevated temperature and humidity (85°C/81% R.H.). After two weeks, the ketal samples were significantly softened. After four weeks, the acetal samples were unchanged (the glass transition temperature and coefficient of thermal expansion were measured by thermomechanical analysis).

### EXAMPLE 9

Removal of the resins derived from the cleavable diepoxides was accomplished by exposing samples of the mixtures cured as in Example 7 to a number of solutions. Cured samples of the mixture containing the Union Carbide ERL-4221 were also exposed at the same time as a control. A solution containing a 1:1:1 volume ratio of glacial acetic acid, ethanol, and deionized water was heated to its boiling point (88°C). The cured ketal diepoxide and the ERL-4221 control were immersed in this solution. After 6 minutes, complete dissolution of the ketal diepoxide had occurred. The control sample was unchanged. Exposure of the cured acetal diepoxide to this solvent showed much slower dissolution--after 60 minutes, the acetal was only partially dissolved. The formal diepoxide was unaffected by this solution.

Similarly, a 135:45:20 ratio of gamma -butyrolactone, water, and phosphoric acid was heated to reflux at 105-106°C. This solvent mixture dissolved the cured ketal diepoxide in < 2 minutes and the cured acetal diepoxide in 10-15 minutes. The control ER-4221 and the formal diepoxide were virtually unaffected.

### EXAMPLE 10

A variety of solvent/acid systems can be used to dissolve the epoxy, and this system may be chosen to minimize attack on the substrate. A system that has proven to be very benign to FR-4 epoxy as well as the copper metallization is a solution of paratoluenesulfonic acid (ptsa) in ethanol. The ethanol acts as both the reactant and the solvent, so no water is required. Ethanol has minimal safety and waste disposal concerns, and ptsa is a solid acid that is easily handled. Acid concentrations of 0.025M to 0.5M have been found to be effective in dissolving the acetal and ketal epoxies. Samples of ketal, similar to those in Example 7, dissolved in well under 10 minutes in a refluxing solution of 0.05M ptsa, while samples of acetal dissolved in 15 minutes in 0.025M ptsa and about 4 minutes in 0.3M ptsa. These acid concentrations are low compared with concentrations of acetic acid (5.8M) or phosphoric acid (1.5M) used previously. While the ptsa/ethanol solutions caused no apparent corrosion of the copper lines, there was still slight attack on the solder balls. The extent of this corrosion was significantly reduced by adding very small amounts of phosphate (as little as .01M) - either as a phosphate salt or phosphoric acid.

## Claims

1. A stable cured diepoxide composition capable of being readily cleaved under specific conditions comprising a ketal or acetal diepoxide having the formula: 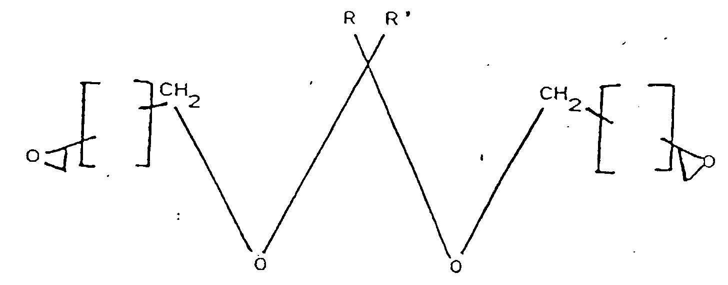 wherein the brackets contain any suitable organic group, the CH₂-groups can optionally be replaced by a bond and R and R' can be any combination of hydrogen, methyl, ethyl, propyl, iso-propyl, butyl, iso-butyl, other alkyl, phenyl, benzyl, substituted phenyl, or substituted benzyl wherein substituents on the phenyl or benzyl can be at one or more of the available aromatic positions and can be a halogen selected from the group consisting of chlorine, bromine, or fluorine, a nitro group, an alkyl group selected from the group consisting of methyl, ethyl, or isopropyl, an alkoxy group selected from the group consisting of methoxy, ethoxy, or isopropoxy, an acyl group selected from the group consisting of acetyl or benzoyl, or an aromatic substituent group; said ketal or acetal diepoxide being exposed to a cyclic anhydride curing agent and an amine promoter being a tertiary amine or an aromatic amine selected from the group consisting of benzyldimethylamine, triethylamine, pyridine, imidazole, propoxylated imidazole, 1,4-diazabicyclo[2.2.2]octane.

2. A composition according to claim 1, wherein the epoxide groups of said diepoxide each being separately connected to a suitable organic group selected from the group consisting of alkylen, cycloalkylen or arylen, and said organic groups each being separately connected to one of the two oxygens of an acyclic acetal or ketal group.

3. The diepoxide composition defined in claim 1, wherein the ketal or acetal diepoxide has the formula: 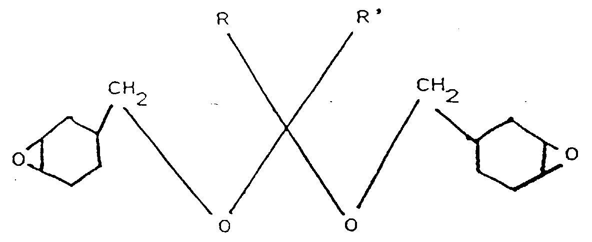 wherein R and R' have the same meaning as in claim 1.

4. The diepoxide composition defined in any one of claims 1 to 3 wherein the cyclic anhydride curing agent is selected from the group consisting of hexahydrophthalic anhydride, methyl-hexahydrophthalic anhydride, nadic methyl anhydride, and maleic anhydride.

5. The diepoxide composition as defined in any one of claims 1 to 4 that contains a flexiblizer material selected from the group consisting of butadiene-acrylonitrile rubber, polyethylene glycol, polypropylene glycol, poly(caprolactone)diol or poly(oxybutylene)diol.

6. The diepoxide composition as defined in any one of claims 1 to 5 that contains an alumina filler or silica filler.

7. The diepoxide composition as defined in claim 6 wherein said filler is silica of particle size 25µ or less and wherein said diepoxide composition contains preferably about 50-70% filler on a weight basis.

8. The diepoxide composition defined in any one of claims 1 to 7 which is acetone bis-(3,4-epoxycyclohexylmethyl) ketal (ketal diepoxide).

9. The diepoxide composition defined in any one of claims 1 to 7 which is acetaldehyde bis-(3,4-cyclohexylmethyl) acetal (acetal diepoxide).

10. The composition of the diepoxide of any one of claims 1 to 9 which encapsulates an electronic substrate, preferably an electronic chip.

11. The method of removing the cured epoxide composition according to anyone of the claims 1 to 9 from an electronic substrate comprising contacting said cured diepoxide encapsulating said substrate with solvent comprising organic solvent, preferably ethanol, gamma-butyrolactone or butanol, acid, or acids, preferably acetic acid, phosphoric acid, paratoluene sulfonic or methane sulfonic acid, and water at 25°C or greater, where, if the solvent is an alcohol, water is dispensable.

12. A structure in which the cured composition of any one of claims 1 to 9 encapsulates and protects an electronic device.

13. The structure of claim 12 in which the electronic device is a silicon integrated circuit electrically connected to a substrate, preferably a ceramic multichip module with solder using controlled collapse chip connection.

14. The structure of claim 13 in which the substrate is a circuit card and in which the cured composition of any one of claims 1 to 9 is used to reinforce the solder connections.

15. The structure of claim 14 in which the electronic device is a ceramic module electrically connected to a substrate with solder balls.

## Patentansprüche

1. Stabile, gehärtete Diepoxidzusammensetzung, die unter speziellen Bedingungen leicht gespalten werden kann und die ein Ketal- oder Acetal-Diepoxid mit der Formel: 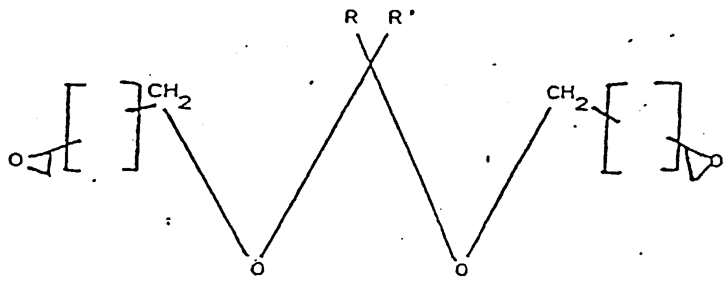 beinhaltet, wobei die Klammern irgendeine geeignete organische Gruppe enthalten, die CH₂-Gruppen optional durch eine Bindung ersetzt werden können und R und R' eine beliebige Kombination aus Wasserstoff, Methyl, Ethyl, Propyl, Isopropyl, Butyl, Isobutyl oder Alkyl, Phenyl, Benzyl, substituiertem Phenyl oder substituiertem Benzyl sein können, wobei Substituenten auf dem Phenyl oder Benzyl auf einer oder mehreren der verfügbaren aromatischen Positionen sitzen können und ein Halogen, das aus der Gruppe ausgewählt ist, die aus Chlor, Brom oder Fluor besteht, eine Nitro-Gruppe, eine Alkylgruppe, die aus der Gruppe ausgewählt ist, die aus Methyl, Ethyl oder Isopropyl besteht, eine Alkoxy-Gruppe, die aus der Gruppe ausgewählt ist, die aus Methoxy, Ethoxy oder Isopropoxy besteht, eine Acyl-Gruppe, die aus der Gruppe ausgewählt ist, die aus Acetyl oder Benzoyl besteht, oder eine aromatische Substituentengruppe sein kann; wobei das Ketal- oder Acetal-Diepoxid einem cyclischen Anhydrid-Härtemittel und einem Aminpromotor ausgesetzt wird, der aus einem tertiären Amin oder einem aromatischen Amin besteht, das aus der Gruppe ausgewählt ist, die aus Benzyldimethylamin, Triethylamin, Pyridin, Imidazol, propoxyliertem Imidazol, 1,4-Diazabicyclo[2.2.2]octan besteht.

2. Zusammensetzung gemäß Anspruch 1, wobei die Epoxidgruppen des Diepoxids jeweils getrennt mit einer geeigneten organischen Gruppe verbunden sind, die aus der Gruppe ausgewählt ist, die aus Alken, Cycloalken oder Arylen besteht, und die organischen Gruppen jeweils getrennt mit einem der zwei Sauerstoffatome einer acyclischen Acetal- oder Ketalgruppe verbunden sind.

3. Diepoxidzusammensetzung, wie in Anspruch 1 definiert, wobei das Ketal- oder Acetaldiepoxid die Formel besitzt: 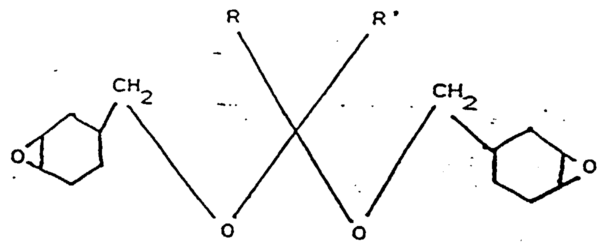 wobei R und R' die gleiche Bedeutung wie in Anspruch 1 haben.

4. Diepoxidzusammensetzung, wie in irgendeinem der Ansprüche 1 bis 3 definiert, wobei das cyclische Anhydrid-Härtemittel aus der Gruppe ausgewählt ist, die aus Hexahydrophtalsäureanhydrid, Methylhexahydrophthalsäureanhydrid, nadischem Methylanhydrid und Maleinsäureanhydrid besteht.

5. Diepoxidzusammensetzung, wie sie in irgendeinem der Ansprüche 1 bis 4 definiert ist, die ein Flexibilisiermaterial enthält, das aus der Gruppe ausgewählt ist, die aus Butadienacrylnitrilgummi, Polyethylenglycol, Polypropylenglycol, Poly(caprolacton)diol oder Poly(oxybutylen)diol besteht.

6. Diepoxidzusammensetzung, wie in irgendeinem der Ansprüche 1 bis 5 definiert, die ein Aluminiumoxid-Füllmaterial oder ein Siliciumdioxid-Füllmaterial enthält.

7. Diepoxidzusammensetzung, wie in Anspruch 6 definiert, wobei das Füllmaterial Siliciumdioxid mit einer Partikelgröße von 25 µm oder weniger ist und wobei die Diepoxidzusammensetzung vorzugsweise etwa 50 % bis 70 % Füllmaterial auf einer Gewichtsbasis enthält.

8. Diepoxidzusammensetzung, wie in irgendeinem der Ansprüche 1 bis 7 definiert, die aus Aceton-bis-(3,4-epoxycyclo-hexylmethyl)ketal (Ketal-Diepoxid) besteht.

9. Diepoxidzusammensetzung, wie in irgendeinem der Ansprüche 1 bis 7 definiert, die aus Acetaldehyd-bis-(3,4-cyclohexylmethyl)acetal (Acetal-Diepoxid) besteht.

10. Zusammensetzung des Diepoxids nach irgendeinem der Ansprüche 1 bis 9, die ein elektronisches Substrat, vorzugsweise einen elektronischen Chip einkapselt.

11. Verfahren zum Entfernen der gehärteten Epoxidzusammensetzung gemäß irgendeinem der Ansprüche 1 bis 9 von einem elektronischen Substrat, welches das Kontaktieren des gehärteten Diepoxids, welches das Substrat einkapselt, mit einem Lösungsmittel, das ein organisches Lösungsmittel, vorzugsweise Ethanol, Gammabutyrolacton oder Butanol, Säure oder Säuren, vorzugsweise Essigsäure, Phosphorsäure, Paratoluolsulfon- oder Methansulfonsäure, und Wasser enthält, bei 25 °C oder mehr beinhaltet, wobei Wasser entbehrlich ist, wenn das Lösungsmittel ein Alkohol ist.

12. Struktur, in der die gehärtete Zusammensetzung nach irgendeinem der Ansprüche 1 bis 9 ein elektronisches Bauelement einkapselt und schützt.

13. Struktur nach Anspruch 12, in der das elektronische Bauelement ein integrierter Schaltkreis aus Silicium ist, der mit einem Substrat, vorzugsweise einem keramischen Mehrfachchipmodul, mittels Lötmittel unter Verwendung der Chipverbindung durch kontrollierten Kollaps verbunden ist.

14. Struktur nach Anspruch 13, in der das Substrat eine Leiterkarte ist und in der die gehärtete Zusammensetzung nach irgendeinem der Ansprüche 1 bis 9 dazu verwendet wird, die Lötmittelverbindungen zu verstärken.

15. Struktur nach Anspruch 14, in der das elektronische Bauelement ein Keramikmodul ist, das mittels Lötmittelkugeln mit einem Substrat elektrisch verbunden ist.

## Revendications

1. Composé diépoxy polymérisé stable capable d'être divisé rapidement sous des conditions spécifiques comprenant un diépoxy cétal ou acétal ayant la formule : 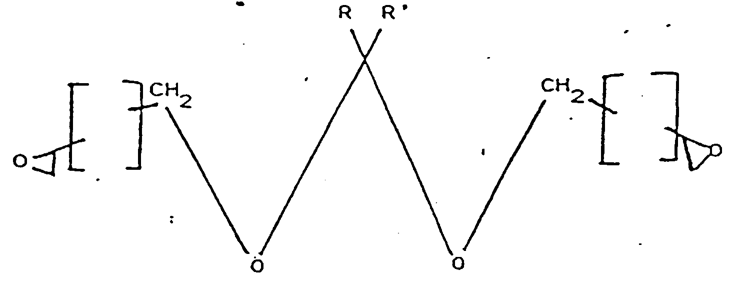 dans laquelle les crochets contiennent tout groupe organique approprié, les groupes CH₂ peuvent, en option, être remplacés par une liaison et R et R' peuvent être toute combinaison d'hydrogène, de méthyle, d'éthyle, de propyle, d'isopropyle, de butyle, d'isobutyle ou autre alkyle, de phényle, de benzyle, de phényle substitué ou de benzyle substitué dans laquelle les substituants du phényle ou du benzyle peuvent se trouver en une ou plusieurs des positions aromatiques disponibles et peuvent être un halogène sélectionné du groupe composé de chlorine, bromine ou fluorine, un groupe nitro, un groupe alkyle sélectionné du groupe composé méthyle, éthyle ou isopropyle, un groupe alkoxyde sélectionné du groupe composé de méthoxyde, éthoxyde ou isopropoxyde, un groupe acyle sélectionné du groupe composé d'acétyle ou de benzoyle, ou un groupe substituant aromatique ; ledit diépoxy cétal ou acétal étant exposé à un agent polymérisant anhydride cyclique et un promoteur aminé du groupe composé de benzyldiméthylamine, triéthylamine, pyridine, imidazole, imidazole propoxylé, 1,4-diazabicyclo[2.2.2]octane.

2. Composé conforme à la revendication 1, dans lequel les groupes époxy dudit diépoxy étant chacun reliés séparément à un groupe organique approprié sélectionné du groupe composé d'alkylène, de cycloalkylèe ou d'arylène, et lesdits groupes organiques étant chacun reliés séparément à l'un des deux oxygènes d'un acétal acyclique ou d'un groupe cétal.

3. Composé diépoxy défini dans la revendication 1, dans lequel le diépoxy cétal ou acétal a la formule : 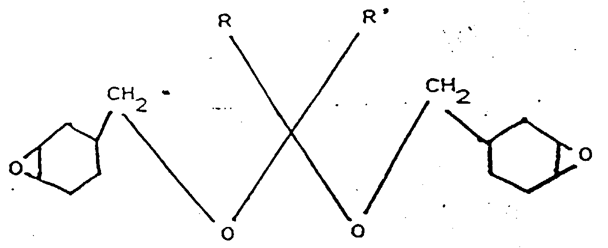 dans laquelle R et R' ont la même signification que dans la revendication 1.

4. Composé diépoxy défini dans l'une quelconque des revendications 1 à 3 dans lequel l'agent polymérisant anhydride cyclique est sélectionné du groupe composé d'anhydride hexahydrophtalique, d'anhydride méthyle-hexahydrophtalique, d'anhydride méthyle n-adique, et d'anhydride maléique.

5. Composé diépoxy tel qu'il est défini dans l'une quelconque des revendications 1 à 4 qui contient un matériau assouplissant sélectionné du groupe composé de caoutchouc butadiène acrylo-nitrile, de polyéthylène glycol, de polypropylène glycol, de poly(caprolactobe)diole ou de poly(oxybutylène)diole.

6. Composé diépoxy tel qu'il est défini dans l'une quelconque des revendications 1 à 5 qui contient une masse de remplissage d'oxyde d'aluminium ou une masse de remplissage de dioxyde de silicium.

7. Composé diépoxy tel qu'il est défini dans la revendication 6 dans lequel ladite masse de remplissage en dioxyde de silicium dont les particules ont une taille de 25 µm ou moins et dans lequel ledit composé diépoxy contient de préférence environ 50 à 70 % de son poids de masse de remplissage.

8. Composé diépoxy défini dans l'une quelconque des revendications 1 à 7 qui est de l'acétone bis-(3,4-époxycyclohexylméthyle)cétal (diépoxy cétal).

9. Composé diépoxy défini dans l'une quelconque des revendications 1 à 7 qui est de l'acétaldéhyde bis-(3,4-cyclohexylméthyle)acétal (diépoxy acétal).

10. Composé du diépoxy de l'une quelconque des revendications 1 à 9 qui enrobe un substrat électronique, de préférence un circuit électronique.

11. Procédé consistant à retirer le composé époxy polymérisé conforme à l'une quelconque des revendications 1 à 9 d'un substrat électronique comprenant la mise en contact dudit diépoxy polymérisé enrobant ledit substrat avec un solvant contenant un solvant organique, de préférence de l'éthanol, du gamma-butyrolactone ou du butanol, de l'acide ou des acides, de préférence de l'acide acétique, de l'acide phosphorique, de l'acide sulfurique paratoluène ou de l'acide sulfurique méthane, et de l'eau à 25 °C ou plus, l'eau étant indispensable si le solvant est un alcool.

12. Structure dans laquelle le composé polymérisé de l'une quelconque des revendications 1 à 9 enrobe et protège un dispositif électronique.

13. Structure de la revendication 12 dans laquelle le dispositif électronique est un circuit intégré au silicium raccordé à un substrat, de préférence un module céramique à plusieurs circuits avec de la soudure en utilisant une connexion du circuit à affaissement contrôlé.

14. Structure de la revendication 13 dans laquelle le substrat est un circuit imprimé et dans laquelle le composé polymérisé de l'une quelconque des revendications 1 à 9 est utilisé pour renforcer les connexions soudées.

15. Structure de la revendication 14 dans laquelle le dispositif électronique est un module céramique relié électriquement à un substrat avec des billes de soudure.
